(19) Europäisches Patentamt
European Patent Office
Office européen des brevets

(11) **EP 4 304 199 A1**

(12) **EUROPEAN PATENT APPLICATION**

(43) Date of publication:
**10.01.2024 Bulletin 2024/02**

(21) Application number: **22183068.0**

(22) Date of filing: **05.07.2022**

(51) International Patent Classification (IPC):
**H04R 1/10** (2006.01)   **H04R 25/00** (2006.01)
**A61B 5/00** (2006.01)

(52) Cooperative Patent Classification (CPC):
**H04R 1/1016; A61B 5/6817; H04R 25/65;**
H04R 2225/025

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Sonova AG**
**8712 Stäfa (CH)**

(72) Inventors:
 • **EL GUINDI, Nadim**
  **8049 Zürich (CH)**
 • **STUMPF, Nina**
  **8708 Männedorf (CH)**
 • **ROECK, Hans-Ueli**
  **8634 Hombrechtikon (CH)**
 • **IGNASIAK, Niklas**
  **8046 Zürich (CH)**

 • **PARKER, Anna**
  **4052 Basel (CH)**
 • **OHS, Nicholas**
  **8046 Zürich (CH)**
 • **RICHNER, Patrizia**
  **8708 Männedorf (CH)**
 • **KUIPERS, Erwin**
  **8633 Wolfhausen (CH)**
 • **SCHLESINGER, Axel**
  **8633 Wolfhausen (CH)**
 • **MOSTAFEI, Maryam**
  **8304 Wallisellen (CH)**
 • **LEUTHOLD, Markus**
  **8712 Stäfa (CH)**
 • **SILBERZAHN, Konstantin**
  **8038 Zürich (CH)**
 • **FEILNER, Manuela**
  **8132 Egg bei Zürich (CH)**

(74) Representative: **Liedtke & Partner Patentanwälte**
**Gerhart-Hauptmann-Straße 10/11**
**99096 Erfurt (DE)**

(54) **EARPIECE**

(57) The invention relates to an earpiece for a hearing device configured to be inserted into an ear canal (8) of a user, the earpiece comprising a housing (1) with a medial end having a sound outlet port (4), the housing (1) accommodating a receiver (20) configured to reproduce sound into the ear canal through the sound outlet port (4), the earpiece further comprising a sensor (2) with a sensing element (11, 12) located on or in a lateral wall of the housing (1), and a retention structure (3) configured to partially cover a surface of the lateral wall of the housing (1), wherein the retention structure (3) comprises a lateral opening leaving a portion of the surface of the lateral wall of the housing (1) uncovered where the sensing element (11, 12) is arranged.

FIG 1A

## Description

Technical Field

The invention relates to an earpiece for a hearing device.

Background of the Invention

Modern in-canal receivers (RIC - receiver in the canal) for hearing aids may be equipped with additional sensors that allow for measurements of physiological parameters of the user. For example, an optical photoplethysmographic sensor (PPG) is used to measures changes in microvascular blood volume, to further extract vital signs such as heart rate, respiration rate, blood oxygenation or blood pressure.

The quality of the PPG signal depends on the placement of the sensor with respect to the tissue to be measured. Consistent and close contact between the light source, the tissue and the photoreceptor ensure optimal penetration of the light through the tissue and efficient recording of the light reflections. In current designs, the RIC is secured in the ear canal by one elastic and symmetric dome at the tip of the RIC. This dome positions the RIC with the PPG sensor approximately central in the ear canal. With no proper attachment of the rear end of the RIC, insufficient contact or even angulation between the sensor and tissue may occur, e.g. due to motion such as walking, chewing and talking, thus compromising the PPG sensor signal quality.

Custom made hearing aid shells, based on individual imprints of the ear canal resolve this issue, create better contact and therefore result in significantly better PPG sensor quality. However, production of customized shells is not only more expensive, but also more cumbersome for the end user as an individual imprint of the ear canal needs to be made.

Summary of the Invention

It is an object of the present invention to provide a solution to improve the signal quality of sensors on an earpiece.

The object is achieved by an earpiece according to claim 1.

Preferred embodiments of the invention are given in the dependent claims.

According to the invention, an earpiece for a hearing device is configured to be inserted into an ear canal of a user, the earpiece comprising a housing with a medial end having a sound outlet port, the earpiece further comprising a sensor with at least one sensing element located on or in a lateral wall of the housing and a retention structure configured to partially cover a surface of the lateral wall of the housing, wherein the retention structure comprises a lateral opening leaving at least a portion of the surface of the lateral wall of the housing uncovered where the sensing element is arranged.

The lateral wall may be a wall extending in parallel to a wall of the ear canal.

In an exemplary embodiment, the sensing element or at least one of the sensing elements may be sensitive to light or to temperature or to a bioelectric signal (e.g. an electrical current or potential generated by a living organism) or may be configured to emit light.

In an exemplary embodiment, the sensor is a photoplethysmography (PPG) sensor or a temperature sensor or a bioelectric sensor (e.g., an electrocardiogram (ECG) sensor, an electroencephalogram (EEG) sensor, an electrooculography (EOG) sensor) or a sensor configured to detect skin impedance. While a PPG sensor may comprise a light source and/or a photo element, a bioelectric sensor may comprise one, two or more electrodes configured to detect a bioelectric signal.

In an exemplary embodiment, a medial end and a distal end of the housing remain uncovered by the retention structure.

In an exemplary embodiment, the retention structure has a sleeve-like shape.

In an exemplary embodiment, the retention structure is removably arranged on the housing.

In an exemplary embodiment, the retention structure is configured to asymmetrically cover the housing.

In an exemplary embodiment, the retention structure is elastic and/or expandable and/or inflatable.

In an exemplary embodiment, the retention structure is made of a biocompatible material, e.g. a foam, a memory foam, silicone, an air-filled cushion and/or consist of or comprises an elastic mesh-like 3D body.

In an exemplary embodiment, the retention structure is configured to optically seal the housing from ambient light when the earpiece is arranged in an ear canal of a user.

In an exemplary embodiment, the retention structure is configured to cover an entire length of the housing.

In an exemplary embodiment, the lateral opening of the retention structure is configured to allow the earpiece to be inserted through the lateral opening.

In an exemplary embodiment, at least one light guide or waveguide is included in the retention structure to increase a signal quality of the sensor.

In an exemplary embodiment, the waveguide is a hollow core or tube-type waveguide.

In an exemplary embodiment, at least one end of the waveguide is covered with a lens or glue or lacquer.

In an exemplary embodiment, the retention structure is arranged on the housing to stabilize the position of the housing in the ear canal without obstructing the sensing element.

In some embodiments, the retention structure is provided at a distance from the sensing element in order to not obstruct the sensing element. In some embodiments, the retention structure is at least partially transparent or translucent to light emitted and/or detectable by the sensing element in order to not obstruct the sensing element. E.g., the retention structure may comprise a waveguide at the position of the sensing element.

The lateral wall may be a wall extending in parallel to a wall of the ear canal.

In an exemplary embodiment, a dome is located on said medial end, e.g. plugged on the sound outlet port, wherein the retention structure is located at a distal end of the earpiece and has one of the following shapes:

- a disk-like shape,
- a dome-like shape,
- a reverse dome shape,
- an oval shape,
- a shape derived from ear space data,
- a wheel with spokes and a rounded rim, and
- a thick non-transparent ring.

The shape of the retention structure may be determined based on ear space data indicative of an ear canal shape of a plurality of users such that the retention structure is configured to conform to an average ear canal shape as derived from the ear space data. For example, the shape of the retention structure may be selected such that the retention structure contacts the average ear canal along a circumference of the ear canal when inserted. For example, the ear space data may be derived from ear impressions taken from the plurality of users.

In an exemplary embodiment, a retention sleeve is arranged over the housing or over a part thereof comprising the distal end, wherein the retention structure is attached to the retention sleeve or wherein the retention sleeve is an integral part of the retention structure.

In an exemplary embodiment, the sensing element or at least one of the sensing elements may be sensitive to light or to temperature or to a bioelectric signal (e.g. an electrical current or potential generated by a living organism) or may be configured to emit light.

In an exemplary embodiment, the sensor is a photoplethysmography (PPG) sensor or a temperature sensor or a bioelectric sensor (e.g., an electrocardiogram (ECG) sensor, an electroencephalogram (EEG) sensor, an electrooculography (EOG) sensor) or a sensor configured to detect skin impedance. While a PPG sensor may comprise a light source and/or a photo element, a bioelectric sensor may comprise one, two or more electrodes configured to detect a bioelectric signal.

In an exemplary embodiment, the shape of the retention sleeve and/or of the retention structure is asymmetrical to bring the sensor, e.g. a PPG sensor or its lights sources and/or photo elements closer to the ear canal.

In an exemplary embodiment, the retention structure is sized to fit into a widened part of the ear canal or into the concha of the user's ear.

In an exemplary embodiment, a soft, asymmetrical retention structure is laterally attached to the housing by a fixture projecting from the retention structure, e.g. the fixture comprising clamp-like prongs or eyes, in which fixture the housing is held.

In an exemplary embodiment, the retention structure is configured as a universal hearing device tip, e.g. such as described in the application EP 21 187 003.5.

In an exemplary embodiment, at least one light guide or waveguide is included in the retention structure and/or in the retention sleeve to increase a signal quality of the sensor.

In an exemplary embodiment, the waveguide is a hollow core or tube-type waveguide.

In an exemplary embodiment, at least one end of the waveguide is covered with a lens or glue or lacquer.

In an exemplary embodiment, the outward end of the waveguide is rounded.

In an exemplary embodiment, the waveguide is provided with a cladding formed from a material with a refractive index of at least 1.4.

In an exemplary embodiment, a cladding of the waveguide for the hearing device tip is made of a soft material with the same mechanical properties as the material of the hearing device tip or made of a stiffer material than the material of the hearing device tip.

In an exemplary embodiment, the cladding may be formed from at least one of an acrylic resin, a silicone, glass or the like.

In an exemplary embodiment, the retention structure is made of a biocompatible material.

In an exemplary embodiment, the retention structure is produced by 2K molding, in particular from a soft material such as silicone or a foam.

The present invention proposes an earpiece or RIC device provided with a sensor, e.g. a PPG sensor, combining the beneficial properties of motion tolerance, good signal quality, easy maintainability (i.e. replaceability of the dome and/or ear wax protection) and immediate availability for the consumer when he enters the store. At the same time, the earpiece or RIC device is easily mass producible in a cost-efficient manner.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

Brief Description of the Drawings

The present invention will become more fully understood from the detailed description given hereinbelow and the accompanying drawings which are given by way of illustration only, and thus, are not limitative of the present invention, and wherein:

Figure 1A   is a schematic view of an exemplary embodiment of an earpiece for a hearing device,

Figure 1B   is a schematic view of another exemplary embodiment of an earpiece for a hearing device,

Figure 2   is another schematic view of the earpiece,

Figure 3   is a schematic view of another exemplary embodiment of an earpiece for a hearing device,

Figure 4   is a schematic view of another exemplary embodiment of an earpiece for a hearing device,

Figure 5   is a schematic view of another exemplary embodiment of an earpiece for a hearing device,

Figure 6   is a schematic view of another exemplary embodiment of an earpiece having waveguides,

Figure 7   is a schematic cross sectional view of the waveguide,

Figure 8   is a schematic longitudinal section of the waveguide,

Figure 9   is a planar single layer reflection model of the waveguide, and

Figure 10   is a schematic view of an example of the waveguide integrated into a universal hearing device tip.

Corresponding parts are marked with the same reference symbols in all figures.

Detailed Description of Preferred Embodiments

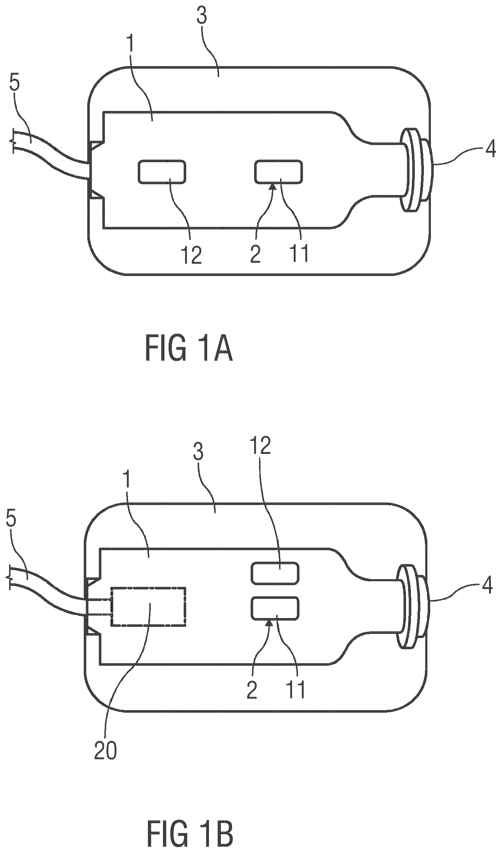

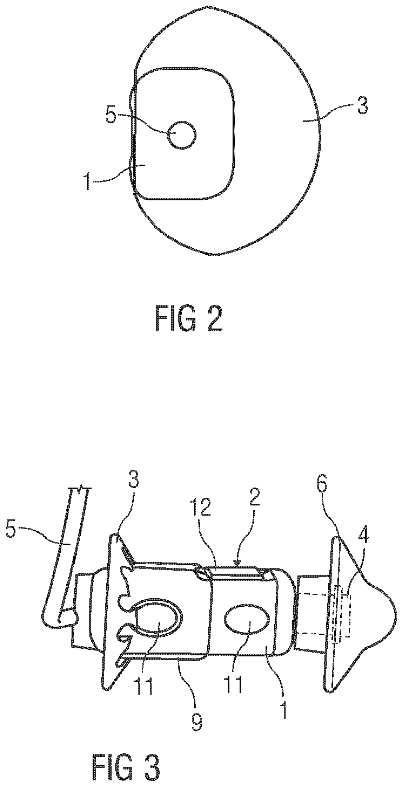

**Figures 1A, 1B and 2** are schematic views of an earpiece or RIC device (receiver in the canal) for a hearing device. The RIC device has a housing 1 having an elongate shape and is configured to be inserted into an ear canal of a user and typically comprises a receiver 20 or speaker configured to reproduce sound into the ear canal through a sound outlet port 4 in a medial surface. The receiver 20 is electrically connected by a cable 5 which enters the housing 1 at a distal end thereof. The cable 5 may also be connected to sound processing circuitry arranged in a behind the ear part of the hearing device. Furthermore, the illustrated RIC device comprises a PPG sensor 2 (photoplethysmographic sensor) comprising at least one sensing element 11 configured as a light source 11 such as an LED 11 and at least one sensing element 12 configured as a photo element 12, e.g. a photo diode 12. The PPG sensor 2 is located on or in a lateral wall of the housing 1 and may also be electrically connected to processing circuitry through the cable 5. The LED 11 and the photo element 12 may be arranged in or on the same side as shown in figure 1A and for example arranged at different longitudinal positions they may be arranged at the same longitudinal position but angularly offset from each other on or in the housing 1 as shown in figure 1B, e.g. by 90° or even by 180°. In principle, the LED 11 and photodetector 12 or the sensing elements 11, 12 in general may be arranged in any configuration, e.g. next to each other along a longitudinal axis extending in parallel to the ear canal, as illustrated in Figure 1A, or at the same axial position but distributed around the circumference, e.g., at 90° or 180° or any other angle, or axially and circumferentially spaced from each other. The lateral wall may face the ear canal wall when the earpiece is at least partially inserted into the ear canal.

In order to improve signal quality of the PPG sensor 2 for the RIC device, a sleeve-like retention structure 3 is arranged to at least partially cover a surface of the lateral wall of the housing 1, whereas a medial end 1.1 and a distal end 1.2 of the housing 1 remain uncovered or do not necessarily have to be covered. The retention structure 3 is configured as a removable and/or replaceable addition to the housing 1 that covers the housing 1 asymmetrically on one side only or on all but one side, leaving a side or multiple sides of the housing 1 at least partially uncovered where the PPG sensor 2 and/or its light source 11 and/or photo diode 12 is arranged. The retention structure 3 thereby ensures optimal or improved fit of the PPG sensor 2 in the ear canal, close contact to the tissue of the ear canal at least on one side and it avoids angulation of the PPG sensor 2, e.g. angular misalignment or angular motion during use. Moreover, the housing 1 may be inserted into the retention structure 3 through a lateral opening thereof which will then leave the side of the housing 1 having the PPG sensor 2 uncovered. The retention structure 3 may be elastic and/or expandable and/or inflatable in order to fit to varying ear canal anatomies and may for example be made of a foam, a memory foam, silicone, air-filled cushions and/or consist of or comprise an elastic mesh-like 3D body.

The elastic properties of the retention structure 3 aim to provide a similar fit of the PPG sensor 2 as can be achieved by wearing a customized shell. However, as the design of the retention structure 3 is based on the design of the housing 1 and yet would fit to different ear canals, it is significantly cheaper in production and easily scalable to a larger group of users.

Additionally, the retention structure 3 may be configured to provide an optical seal, in order to prevent outside light entering the ear canal and thereby interfering with the PPG measurement.

In some embodiments, only one of the LED 11 and the photodetector 12 is located at the uncovered side and another one is provided at a covered side, at which a light transparent window and/or a waveguide may be implemented in the retention structure 3. Alternatively, the retention structure 3 may be made of a light transparent material.

The described solution ensures optimal contact between PPG sensor 2 and the tissue in the ear canal and thereby improves PPG signal quality.

The same isolation principle may apply to RIC devices with different types of sensors, e.g. for temperature measurement, to enable the RIC device to assess a core body temperature.

**Figure 3** is a schematic view of an earpiece or RIC device (receiver in the canal) for a hearing device. The RIC device has a housing 1 having an elongate shape and is configured to be inserted into an ear canal of a user and typically comprises a receiver or speaker (not shown) configured to reproduce sound into the ear canal through a sound outlet port 4 in a medial surface. The receiver is electrically connected by a cable 5 which enters the housing 1 at a distal end thereof. The cable 5 may also be connected to sound processing circuitry arranged in a behind the ear part of the hearing device. Furthermore, the illustrated RIC device comprises a sensor, e.g. a PPG sensor 2 (photoplethysmographic sensor) comprising at least one sensing element 11 configured as a light source 11 such as an LED 11 and at least one sensing element 12 configured as a photo element 12, e.g. a photo diode 12. The PPG sensor 2 is located in or on a lateral surface of the RIC device 1 and may also be electrically connected to processing circuitry through the cable 5.

At the medial end of the housing 1, a dome 6 is arranged. The dome 6 may be plugged onto the sound outlet port 4 (also referred to as speaker output or spout) of the housing 1 to provide acoustic sealing within the ear canal in order to prevent sound emitted from the sound outlet port 4 to be transmitted to a microphone (not shown in detail) of the hearing device. Further, the dome 6 may be adapted to properly fit the housing 1 to the ear canal of the user.

Different domes 6 may vary in outer shape, size, material, elasticity, etc. The dome 6 may be configured to be plugged or clipped onto the sound outlet port 4.

A retention structure 3 may be configured to provide an optical seal, in order to prevent outside light entering the ear canal and thereby interfering with the PPG measurement. Moreover, an additional acoustic damping may be achieved. To keep a cerumen protection function separate from a stabilization function, a retention structure 3, e.g. a further dome-like structure, may be located at the distal end of the housing 1. The retention structure 3 may be mounted on one or more knobs (not shown) on the housing 1 which could alternatively be used to mount a retention rod. A retention sleeve 9 may be arranged over the housing 1 or over a part thereof comprising the distal end. Instead of the rod, the retention structure 3 may be attached to the retention sleeve 9 and/or to the housing 1. In the latter case the retention sleeve 9 may be an integral part of the retention structure 3. In the illustrated example, the retention structure 3 is an essentially disk shaped dome. Alternative shapes may include:

- a reverse dome shape
- an oval shape

- a structure including a venting channel extending through the structure to provide for a venting between an inner region of the ear canal and the ambient environment outside the ear canal". Alternatively, the structure may have no venting channel, thus providing for an acoustically closed fitting of the RIC devicea shape inspired by ear space data (universal tip cross section)
- a wheel with spokes and rounded rim
- a thick substantially non-transparent ring blocking ambient light

The retention structure 3 serves as a fixture to reduce free movement of the RIC device in the ear canal, at least with regard to a longitudinal axis of the RIC device. The retention structure 3 is arranged at a distal end of the housing 1 in order not to obstruct and/or disturb a light path of the PPG sensor 2 which is preferably located on or in a lateral surface of the housing 1 between the medial end and the distal end thereof. The retention structure 3 may be provided with different geometries to fit the left and the right ear canal. Moreover, the retention structure 3 may be provided in different sizes to accommodate different ear geometries. For example, the RIC device may be provided as a kit with several replaceable retention structures 3 of different size. The retention structure 3 may preferably be made of a biocompatible material. In an exemplary embodiment, at least one light or waveguide may be included in the retention sleeve 9 to increase signal quality of the PPG sensor 2. In an exemplary embodiment, the shape of the retention sleeve 9 and/or of the retention structure 3 may be asymmetrical to bring diodes and sensor closer to the ear canal while maintaining wearing comfort.

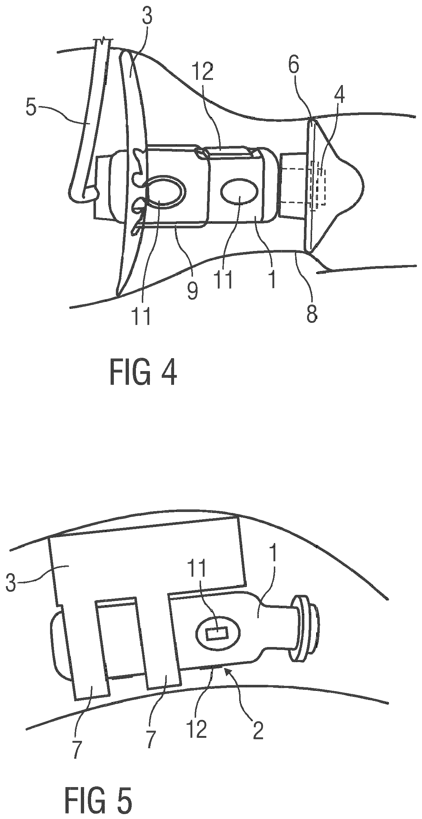

**Figure 4** is a schematic view of an exemplary embodiment of an earpiece or RIC device having a retention structure 3 which is larger and shaped differently from the one of figure 3. Also shown is the ear canal 8. The retention structure 3 is enlarged to fit into a widened part of the ear canal 8 or into the concha of the user's ear.

**Figure 5** is a schematic view of a housing 1 of an earpiece or RIC device, to which a soft, asymmetrical retention structure 3 is laterally attached, e.g. by clamp-like prongs 7 or eyes 7, in which the housing 1 may be held. The asymmetrical shape brings the sensor 2, e.g. the PPG sensor 2, closer to the wall of the ear canal 8 similar to the embodiment of figures 1A, 1B and 2, increasing the signal quality by reducing the extent of relative movement possible between the sensor 2 and the ear canal 8 and by shortening the light path of light emitted by the sensor 2 or its light sources 11. The shape of the retention structure 3 shown in figure 5 is simplified and appears rectangular. However, the shape of the retention structure 3 should rather be rounded, e.g. modelled similar to universal hearing device tips or earpieces, e.g. using ear space data as described in the application EP 21 187 003.5, which is hereby incorporated by reference into the present application in its entirety.

In an exemplary embodiment, a solid or hollow core or tube-type waveguide 10 may be incorporated in the universal hearing device tip or retention structure 3 or retention sleeve 9.

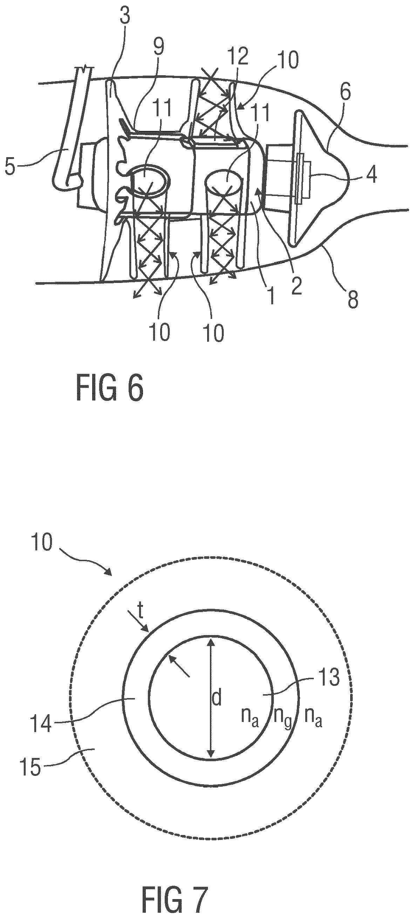

**Figure 6** is a schematic view of an earpiece or RIC device (receiver in the canal) for a hearing device similar to the one shown in figures 3 and 4, but additionally comprising respective waveguides 10 for the at least one light source 11 of the PPG sensor 2 and/or for the photo diode 12 of the PPG sensor 2. The waveguides 10 may extend in an essentially radial direction relative to the housing 1. The outward ends of the waveguides 10 may be covered with a respective thin lens or glue or lacquer or another suitable substance to provide ingress protection against earwax. In an exemplary embodiment, a machine-based lacquering process may be used to prevent unwanted optical loss due to the shape of the lacquering layer. The outward ends of the waveguides 10 may be rounded to protect the ear canal 8 from rashes and/or itching. As illustrated, the waveguides 10 may be implemented in the retention sleeve 9.

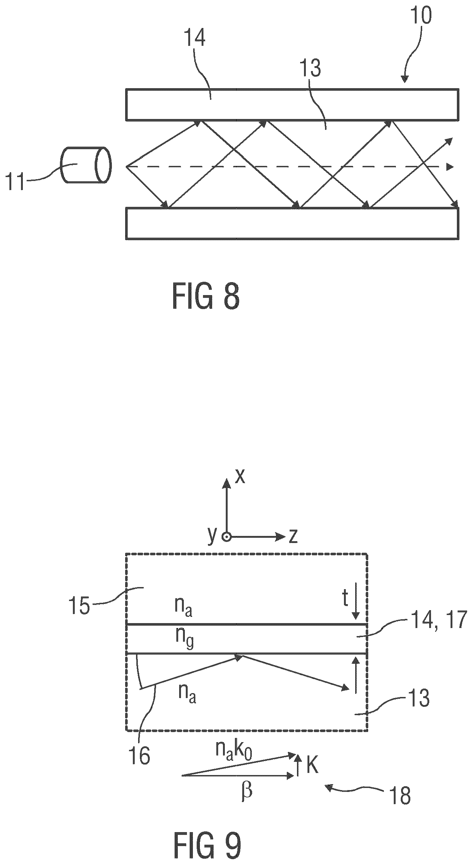

**Figure 7** is a schematic cross sectional view of the waveguide 10. **Figure 8** is a schematic longitudinal section of the waveguide 10. **Figure 9** is a planar single layer reflection model of the waveguide 10.

The waveguide 10 serves for enhancing the signal to noise ratio of the measurements made by the sensor 2, e.g. the PPG sensor 2.

Hollow-core optical waveguides 10 are configured to guide light in a core 13 inside a micro-structured cladding 14 or tube and are capable of guiding light to and from tissue with very low dispersion and/or optical loss as compared to solid waveguides. Many of the limitations of conventional optical waveguides originate from the fact that light propagates in solid high refractive mediums such as glass and cannot be guided effectively.

In the presently proposed design, the core 13 is an air core surrounded by a micro-structured dielectric cladding 14 (or ring or tube) that confines the light to the core 13. In an exemplary embodiment, the waveguide 10 is a tube-type anti-resonant hollow core waveguide, comprising the air core 13 within a high refractive medium cladding 14 (e.g. made of glass, silicone, acrylic resin, etc.), and an air cladding 15 surrounding the cladding 14. The air within and outside the cladding 14 has a refractive index $n_a$ the cladding has a refractive index $n_g$.

The cladding 14 may have a reflective surface or coating.

In order to analyse the guidance conditions in the tube-type optical waveguide 10 the structure is considered as an anti-resonant reflecting optical waveguide. As a double-layered Fabry-Perot resonator, there are anti-resonant wavelengths where constructive interference occurs inside the cladding 14 supporting the light transmission. At resonant wavelengths, the light couples to lossy modes inside the dielectric cladding 14. This guidance mechanism is known as the anti-resonant effect with inhibited coupling with low loss and wideband transmission.

The planar single layer reflection model of figure 9 shows a single wave 16 reflected on a planar glass film 17 from an interior air side, e.g. the core 13, where x is a direction perpendicular to the planar glass film 17, y is a direction parallel to the planar glass film 17 but transverse with regard to the propagation of a wave 16, and z is a direction parallel to the planar glass film 17 and essentially in parallel with the parallel with the propagation of the wave 16. A wave vector diagram 18 of the propagating mode, i.e. of the wave 16 incident on the glass film 17 is shown below the single layer reflection model, wherein $\beta$ is a propagation constant, nako is the wave vector of the incident wave 16, and K is the transverse wave vector.

The main approximation of the waveguide 10 is that a diameter d of the core 13 is much larger than an operational wavelength $\lambda$ ($d \gg \lambda$). In this design, it is possible to confine light in the core 13 over broadband spectral regions aside from where resonances with the cladding 14 occur. The spectral position of these resonances in the cladding 14 (ring) can be calculated by a simple expression,

$$\lambda_m = \frac{2t}{m}\sqrt{n^2 - 1}, m = 1, 2, 3 \ldots$$

where t is the thickness of the cladding 14 (ring), n is the refractive index of the cladding 14 (ring) and m is an integer value indicating the order of the resonance.

The resonances are evenly spaced in frequency, and between them anti-resonant windows open in which the waveguide 10 transmits light with low loss.

In an exemplary embodiment, the core 13 diameter d may be in the range of 1 mm to 2 mm, e.g. 1 mm. The thickness t of the cladding 14 may be in the range of 0.1 mm to 0.5 mm, e.g. 0.2 mm. In this case, resonance may occur at $\lambda$=447 $\mu$m, 112$\mu$m, 49.7 $\mu$m etc. and anti-resonance at $\lambda/2$. The optical loss at 840 nm (infrared) would be about 2.5%. As opposed to this with a cladding 14 thickness t of 0.7 mm, the optical loss at 840 nm (infrared) would be about 31%.

In this model, the high refractive index layer (ring or cladding 14) is regarded as a Fabry-Perot cavity. The light goes out of the high refractive index layer when the wavelength is close to the resonant wavelength. However, when the propagating wavelength is far away from the resonant wavelength, the light should be reflected, resulting in good confinement. Therefore, the matching condition of wavelength determines the band edges of high-loss and low loss regions.

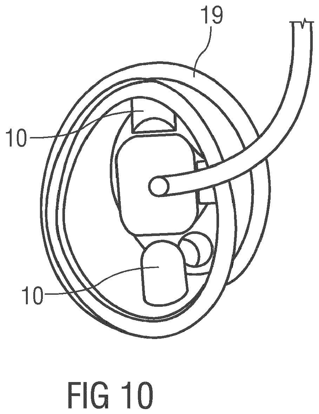

**Figure 10** is a schematic view of an example of the hollow core optical waveguide 10 integrated into a universal hearing device tip 19 (soft or hard), e.g. such as in EP 21 187 003.5. The waveguide 10 for the hearing device tip 19 may be made of a soft material with the same mechanical properties as the hearing device tip 19 or it may be made of a soft material with a lower softness (i.e. stiffer) than the hearing device tip 19. The ends of the waveguides 10 may be covered with a thin lens or glue or lacquer or another suitable substance to provide ingress protection against earwax. In an exemplary embodiment, a machine-based lacquering process may be applied to prevent unwanted optical loss due to the shape of lacquering layer.

As usual, the hearing device (or ear level device in general to include also cochlear implants, earbuds etc.) contains a battery powering the system, the battery preferentially being of a rechargeable type, such as a Li-Ion cell. It further contains a power management unit, at least one microphone, one or multiple processors processing the microphone signals as well as the PPG sensor signal and a motion sensor signal, producing sound output signals being emitted by the receiver into the ear canal 8 of the user. Processed PPG signals may be transmitted by a wireless transceiver to a further device. The processing of the microphone signal may consist of, among others, amplification for hearing loss compensation, noise cancellation, feedback cancellation, beamforming, limitation of the maximal sound level, classification of the sound types and/or adaptation to individually preferred sound settings. The processor may alternatively or in addition decode a sound signal as received from the wireless transceiver.

The processing of the PPG sensor signal may consist of movement artefact removal using the motion sensor signal, extraction of physiologic parameters such as heart rate, respiration rate, heart rate variability, blood pressure, any blood analyte level such as Sp02, blood glucose, hydration or any other such relevant physiologic parameter. Sensor signals from the microphone, PPG sensor, motion sensor, temperature sensor etc. and/or processed versions thereof might be sent to an external device for further processing, visualization, storage and distribution.

In an exemplary embodiment, the retention structure 3 may be produced by 2K molding, in particular from a soft material such as silicone or a foam.

This presently disclosed solutions may reduce movement of the sensor 2 relative to the ear canal 8. The additional mechanical fixation prevents the sensor 2 from moving mechanically relative to the human ear canal 8, which would interfere and add noise to the PPG measurement. The fixation also blocks extraneous light entering the body from the sun or other external sources, thus preventing foreign light from saturating the PPG sensor 2.

The skilled person readily understands that any feature of any of the described embodiments may be applied in any of the other embodiments.

List of References

1　　housing

| 2 | sensor, PPG sensor |
|---|---|
| 3 | retention structure |
| 4 | sound outlet port |
| 5 | cable |
| 6 | dome |
| 7 | fixture, prong, eye |
| 8 | ear canal |
| 9 | retention sleeve |
| 10 | waveguide |
| 11 | sensing element, light source, LED |
| 12 | sensing element, photo element, photo diode |
| 13 | core |
| 14 | cladding |
| 15 | air cladding |
| 16 | wave |
| 17 | planar glass film |
| 18 | wave vector diagram |
| 19 | hearing device tip |
| 20 | receiver |

| d | diameter |
|---|---|
| K | transverse wave vector |
| na | refractive index |
| nako | wave vector |
| $n_g$ | refractive index |
| t | thickness |
| x | direction |
| y | direction |
| z | direction |
| β | propagation constant |

**Claims**

1. An earpiece for a hearing device configured to be inserted into an ear canal (8) of a user, the earpiece comprising a housing (1) with a medial end having a sound outlet port (4), the housing (1) accommodating a receiver (20) configured to reproduce sound into the ear canal through the sound outlet port (4), the earpiece further comprising a sensor (2) with a sensing element (11, 12) located on or in a lateral wall of the housing (1) and a retention structure (3) configured to partially cover a surface of the lateral wall of the housing (1), wherein the retention structure (3) comprises a lateral opening leaving a portion of the surface of the lateral wall of the housing (1) uncovered where the sensing element (11, 12) is arranged.

2. The earpiece of claim 1, wherein the sensing element (11, 12) is sensitive to light or to temperature or to a bioelectric signal or the sensing element (11, 12) is configured to emit light.

3. The earpiece of claim 1 or 2, wherein a medial end (1.1) and a distal end (1.2) of the housing (1) remain uncovered by the retention structure (3).

4. The earpiece according to any one of the preceding claims, wherein the retention structure (3) has a sleeve-like shape.

5. The earpiece according to any one of the preceding claims, wherein the retention structure (3) is removably arranged on the housing (1).

6. The earpiece according to any one of the preceding claims, wherein the retention structure (3) is configured to asymmetrically cover the housing (1).

7. The earpiece according to any one of the preceding claims, wherein the retention structure (3) is elastic and/or expandable and/or inflatable.

8. The earpiece according to any one of the preceding claims, wherein the retention structure (3) is made of a biocompatible material.

9. The earpiece according to any one of the preceding claims, wherein the retention structure (3) is made of a foam, a memory foam, silicone, an air-filled cushion and/or consist of or comprises an elastic mesh-like 3D body.

10. The earpiece according to any one of the preceding claims, wherein the retention structure (3) is configured to optically seal the housing (1) from ambient light.

11. The earpiece according to any one of the preceding claims, wherein the retention structure (3) is configured to cover an entire length of the housing (1).

12. The earpiece arrangement according to any one of the preceding claims, wherein the lateral opening of the retention structure (3) is configured to allow the earpiece to be inserted through the lateral opening.

13. The earpiece arrangement according to any one of the preceding claims, wherein at least one light guide or waveguide (10) is included in the retention structure (3) to increase a signal quality of the sensor (2).

14. The earpiece arrangement according to claim 13, wherein the waveguide (10) is a hollow core or tube-type waveguide (10).

15. The earpiece arrangement according to claim 13 or 14, wherein at least one end of the waveguide (10) is covered with a lens or glue or lacquer.

FIG 1A

FIG 1B

FIG 2

FIG 3

FIG 4

FIG 5

FIG 6

FIG 7

FIG 8

FIG 9

FIG 10

Europäisches
Patentamt

European
Patent Office

Office européen
des brevets

# EUROPEAN SEARCH REPORT

Application Number

EP 22 18 3068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/196073 A1 (NIELSEN HENRIK [DK] ET AL) 18 June 2020 (2020-06-18) | 1-5,7-9, 11 | INV. H04R1/10 |
| A | * paragraphs [0054] - [0057]; figures 1,2 * | 6,12-15 | H04R25/00 A61B5/00 |
| X | US 2019/253793 A1 (PEDERSEN TROELS HOLM [DK] ET AL) 15 August 2019 (2019-08-15) | 1-9,11, 12 | |
| A | * paragraphs [0294] - [0298]; figure 34D * | 13-15 | |
| X | US 2021/085254 A1 (WANG FRANK [US] ET AL) 25 March 2021 (2021-03-25) | 1-5,7-10 | |
| A | * paragraphs [0014] - [0021]; figures 3,6-9 * | 13-15 | |

| TECHNICAL FIELDS SEARCHED (IPC) |
|---|
| H04R A61B |

The present search report has been drawn up for all claims

| Place of search | Date of completion of the search | Examiner |
|---|---|---|
| The Hague | 8 February 2023 | Radomirescu, B-M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

Europäisches
Patentamt

European
Patent Office

Office européen
des brevets

**Application Number**

**EP 22 18 3068**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

16

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

      **1. claims: 1-9, 11, 12**

           **Earpiece having an asymmetrical retention structure**
                    ---

      **2. claim: 10**

           **Earpiece having optical sealing retention structure**
                    ---

      **3. claims: 13-15**

           **Earpiece having light guides or waveguides**
                    ---

## ANNEX TO THE EUROPEAN SEARCH REPORT
## ON EUROPEAN PATENT APPLICATION NO.

EP 22 18 3068

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2023

| Patent document cited in search report | | Publication date | Patent family member(s) | | Publication date |
|---|---|---|---|---|---|
| US 2020196073 | A1 | 18-06-2020 | CN | 111327982 A | 23-06-2020 |
| | | | EP | 3672272 A1 | 24-06-2020 |
| | | | JP | 2020120372 A | 06-08-2020 |
| | | | US | 2020196073 A1 | 18-06-2020 |
| US 2019253793 | A1 | 15-08-2019 | CN | 110166916 A | 23-08-2019 |
| | | | CN | 114827860 A | 29-07-2022 |
| | | | EP | 3525490 A1 | 14-08-2019 |
| | | | US | 2019253793 A1 | 15-08-2019 |
| | | | US | 2021105558 A1 | 08-04-2021 |
| | | | US | 2022256282 A1 | 11-08-2022 |
| US 2021085254 | A1 | 25-03-2021 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 21187003 A